# EUROPEAN PATENT SPECIFICATION

(11) **EP 3 741 293 B1**
(45) Date of publication and mention of the grant of the patent: **30.06.2021**
(21) Application number: 19176395.2
(22) Date of filing: 24.05.2019
(51) Int. Cl.: A61B 5/026, G01R 33/56, G01R 33/565, A61B 5/055, A61B 5/113

(54) **METHOD AND SYSTEM FOR MEASURING BLOOD FLOW**
VERFAHREN UND SYSTEM ZUR MESSUNG DER DURCHBLUTUNG
PROCÉDÉ ET SYSTÈME POUR MESURER LE DÉBIT SANGUIN

(43) Date of publication of application: 25.11.2020
(73) Proprietor: Siemens Healthcare GmbH, 91052 Erlangen (DE)
(72) Inventor: Bacher, Mario, 91052 Erlangen (DE); Speier, Peter, 91056 Erlangen (DE)

(56) References cited:
- EP-A1- 3 413 076
- WO-A1-2019/096707
- US-A1- 2010 106 008
- US-A1- 2015 241 538
- US-A1- 2015 272 453
- US-A1- 2017 160 364
- US-A1- 2018 055 380
- US-A1- 2018 353 139

## Description

The invention describes a method and a system for measuring blood flow, especially for a magnetic resonance imaging system ("MRI system").

MRI systems are used in medical examinations for recording (imaging) data of an examination object by exciting its nuclear spins that are aligned within a strong basic magnetic field. The precession or relaxation of the spins from this excited state into a state with less energy generates, as a response, a magnetic alternating field (radio frequency signal, "RF signal") that is received via RF antennae. The RF frequency strongly depends on the basic magnetic field.

Depending on the pulse sequence used for recording, the measurement of the MRI system requires a number of milliseconds up to seconds. While a longer recording time usually results in minimal noise artifacts, the influence of motion artifacts increases with the duration of the measurement.

Although most patients try to remain still, in order to avoid motion artifacts, there are unavoidable movements of the patient that cannot be stopped, such as e.g. breathing or heartbeat.

It is state of the art to measure the movements of a patient by using mechanical sensors or electrodes that measure the excitation potential of the muscles.

German patent application 10 2015 203 385 A1 describes a basic method of capturing the movements using a high-frequency signal. The signal is permanently captured in a patient recording of a magnetic resonance tomography system, and signal changes due to movements (e.g., due to changing interferences or damping) are evaluated. A movement of the patient caused by breathing or heartbeat may then be identified from certain patterns of this signal.

German patent application 10 2015 224 158 A1 discloses a special transmitter for pilot tone navigation in a magnetic resonance tomography system. This pilot tone navigator includes a power supply and an antenna. The transmitter is configured to emit a pilot tone signal in the form of an electromagnetic alternating field by way of the antenna. The transmitter includes a decoupling element that decouples the transmitter output of signals that the antenna in a magnetic resonance tomography system receives by excitation pulses of the magnetic resonance tomography system.

German patent application 10 2015 224 158 A1 further discloses a method of identifying a movement of a patient using a magnetic resonance tomography system with such transmitter that is arranged in close proximity to the heart or the lungs of the patient (e.g., on a body surface of the patient at a minimal distance from the organs). Then, the transmitter transmits the pilot tone signal and the magnetic resonance tomography system receives the pilot tone signal with one or a number of antennae and receivers that are typically provided to receive a magnetic resonance signal.

Although it is possible to measure the motion of the heart or of the lungs, no non-invasive sensor currently exists that is capable of continuously detecting flow within a magnetic resonance ("MR") scanner environment. This is very disadvantageous, since the accurate detection of blood flow is an important factor in non-contrast MR angiography (NC-MRA) like NATIVE or QISS of extremities where the correct trigger time, i.e. the arrival of the pulse wave in the examination volume, is delayed with respect to the ECG R-peak by an unknown time.

The unknown time delay between contraction of the heart and arrival of the pulse wave in the imaging volume is up to now either estimated based on length measurements or trial scans, or free-running sequences are used to measure continuously.

Currently existing magnetic flowmeters for blood flow use electrodes attached to the skin to measure induced potentials due to motion US-2015/272453-A1, US-2015/241538-A1 and US-2018/055380-A1 show MRI angiography systems. WO-2019/096707-A1, EP-3413076-A1, US-2010/106008-A1, US-2018/353139-A1, and US-2017/160364-A1 show methods of motion detection in MRI systems.

It is the object of the present invention to improve the known systems, devices, and methods to facilitate an improvement for measurements of a blood flow of a patient, especially with a magnetic resonance imaging system.

This object is achieved by a method according to claim 1, a system according to claim 8, a control device according to claim 12, and a magnetic resonance imaging system according to claim 13.

A method according to the invention can be used for measuring blood flow. This blood flow could typically be the blood flow in an object of a patient, wherein the object is typically a blood vessel, but could also be regarded as an organ comprising blood vessels or another cavity where blood may flow (also a wound). The patient is preferably positioned in a magnetic resonance imaging system.

The method comprises the following steps:
- Generating a (low-power) modulated magnetic field by a generator coil. This field should comprise at least one frequency that can be measured by an RF reception system. A simple modulated magnetic field could have the shape of a sine wave. However, it is also possible to have a more complex modulation comprising a multiplicity of sine waves, e.g. a rectangular modulation. Since it is very advantageous in magnetic resonance imaging to use the RF reception system of a magnetic resonance scanner, the frequency (or at least one dominant frequency) should accord to the receiving bandwidth of this RF reception system but deviate from the Larmor frequency recorded during regular data acquisition for imaging. It is clear that best results are achieved when the modulated magnetic field is sent directly to the object positioned for examination.

- Recording response signals of the modulated magnetic field from the object with the RF reception system, wherein the response signals are measured by at least two RF antennae of the RF reception system. These RF antennae are preferably coils, particularly preferably coils used for imaging in a magnetic resonance scanner, however, they could also have another shape (e.g. of a linear dipole).
- Separating a flow component of the recorded response signals from those signal components resulting from movements of the object. Such movements could include, besides muscular movements of the extremities, cardiac and respiratory motion or bowel movements. A movement-dependent change in the received signal can be identified and then separated by using signal analysis. For example, a flow movement may be detected by identifying a variation in the amplitude of the modulated magnetic field ("pilot tone signal"), caused by changes in the load of the RF antennae or the change in superimpositions or damping due to the blood flow in a blood vessel. The flow movement may be differentiated from other disturbing influences by the specific frequency and signal profile that is different from the frequency or signal profile of e.g. a respiratory movement or a heartbeat.

A system for measuring blood flow is preferably designed to perform a method according to the invention and comprises the following components:
- A first data-interface designed for sending a signal to a generator coil, wherein this signal is designed to generate a (low-power) modulated magnetic field in the generator coil. This signal could be generated by a signal generator that can also be part of the system. However, the signal generator could also be part of an (external) generator coil, wherein the signal sent through the data interface could be a simple "on" signal to trigger the signal generator.
- A second data-interface designed for recording response signals of the modulated magnetic field from an object measured with an RF reception system, wherein at least the response signals are measured by two RF antennae of the RF reception system. The RF reception system could be part of the system and should comprise data acquisition units that are designed to modify the signals of the RF antennae, e.g. amplify and/or shape it, such that the system can use the signals for further processing. For example, the RF reception system can provide digital data of the recorded signals. It should be noted that the first and second data interface could be one single unit designed for bi-directional communication.
- A separation unit designed to separate a flow component of the recorded response signals from those signal components resulting from movements of the object.

The whole setup with the system comprising the generator coil RF reception system could be designated as "Pilot Tone navigator system" since it uses a pilot "tone" (the modulated magnetic field) for measurements. Changes in the electromagnetic environment within the examination volume, e.g. by blood flow, respiratory motion, cardiac motion or further muscular or bowel movements, alter the generated modulated magnetic field. When the generative field is set to a frequency just outside the imaging bandwidth around the Larmor frequency, the modulated magnetic field can easily be detected by the RF antennae.

The generator coil may comprise a transmitter for pilot tone navigation in a magnetic resonance tomography system. Preferably, the generator coil generates a modulated magnetic field with a first frequency band. Preferably, an excitation signal is generated by an RF transmission antenna system of a magnetic resonance imaging system to generate a magnetic resonance signal with a second frequency band from the object. Preferably, the second frequency band is at least essentially outside the first frequency band. Preferably, the least two RF antennae of the RF reception system is configured to receive a frequency band that includes the first frequency band and the second frequency band.

Since blood is a (para)magnetic fluid, the modulated magnetic field induces eddy currents in the flowing blood, which in turn generates a magnetic field opposing the inducing field. Thus, when the blood moves due to the heart's pumping activity, an additional current is induced due to this motion. This changes the local magnetic environment and the response would differ from the original "tone", i.e. the original modulation of the initial magnetic field.

Since the response is measured using a (e.g. relatively vast) number of RF antennae (e.g. receiver coils of an MR scanner), the flow component can be separated from those induced from cardiac and respiratory motion or bowel/muscular movements using appropriate blind and/or semi-blind source separation methods such as e.g. Independent Component Analysis (ICA) or training with ground truth data.

A control device according to the invention for controlling a magnetic resonance imaging system comprises a system according to the invention and/or is designed to perform a method according to the invention. The control device may comprise additional units or devices for controlling components of a magnetic resonance imaging system, e.g. a sequence control unit for measurement sequence control, a memory, an RF transmission device that generates, amplifies, and transmits RF pulses, a gradient system interface, an RF reception device to acquire magnetic resonance signals and/or a reconstruction unit to reconstruct magnetic resonance image data.

A magnetic resonance imaging system according to the invention comprises a control device according to the invention or is at least designed to perform a method according to the invention.

Some units or modules of the system or the control device mentioned above can be completely or partially realized as software modules running on a processor of a system or a control device. A realization largely in the form of software modules can have the advantage that applications already installed on an existing system can be updated, with relatively little effort, to install and run these units of the present application. The object of the invention is also achieved by a computer program product with a computer program that is directly loadable into the memory of a device of a system or a control device of a magnetic resonance imaging system, and which comprises program units to perform the steps of the inventive method when the program is executed by the control device or the system. In addition to the computer program, such a computer program product can also comprise further parts such as documentation and/or additional components, also hardware components such as a hardware key (dongle etc.) to facilitate access to the software.

A computer readable medium such as a memory stick, a hard-disk or other transportable or permanently-installed carrier can serve to transport and/or to store the executable parts of the computer program product so that these can be read by a processor unit of a control device, an MPSU or a system. A processor unit can comprise one or more microprocessors or their equivalents.

Particularly advantageous embodiments and features of the invention are given by the dependent claims, as revealed in the following description. Features of different claim categories may be combined as appropriate to give further embodiments not described herein.

According to a preferred method, the generator coil is embedded in a magnetic resonance scanner of a magnetic resonance imaging system, preferably near or in an RF reception antenna system (with one or more RF antennae) of the magnetic resonance scanner. It is preferred that the generator coil is positioned adjacent the examination space or in a unit that is placed at, under or on a patient for examination, e.g. in a local coil setup, since the nearer the generator coil is positioned to the patient the greater the excitation of the desired area with the modulated magnetic field. It is preferred that a number of coils used for imaging in a magnetic resonance scanner are used as generator coils.

According to a preferred method, the RF reception system used for recording the response signals of the modulated magnetic field is the RF reception system of a magnetic resonance imaging system, wherein preferably the response signals of the modulated magnetic field are recorded by RF antennae from the RF reception antenna system of the magnetic resonance scanner. As stated above, it is preferred that the, or at least one, (dominant) frequency of the modulation of the magnetic field lies within the normal bandwidth of the RF reception antenna system of the magnetic resonance scanner (e.g. in the order of some ten or hundred MHz). The frequency is dependent of the field strength, e.g. at a basic magnetic field of 7 T the frequency could be close to 300 MHz, at field strength below 0.5 T the frequency could be lower than 20 MHz. At a basic magnetic field of 1.5 T the frequency could be especially over 22 MHz and/or lower than 150 MHz. For example, since the Larmor frequency at a basic magnetic field of 1.5 T is about 62.5 MHz, the frequency used for modulation may be between 57 MHz and 61 MHz or 64 MHz and 68 MHz. It is preferred that more than three RF antennae are used for measurement, since the more antennae are used the better the separation of the response signals can be performed.

With the Larmor frequency for a proton with the gyromagnetic moment of 267,5 MHz/T, the Larmor frequency fL at a magnetic field B could be calculated from fL = 267,5/(2·π)·B MHz/T. Thus, the frequency f used for modulation at a present magnetic field B should be around (but especially not equal) fL = 42,58·B MHz/T. It is preferred that the frequency f used for modulation at a present magnetic field B is below 42·B MHz/T and above 43·B MHz/T, however, It is particularly preferred that the frequency f used for modulation at a present magnetic field B is above 38·B MHz/T (if lower than the Larmor frequency) or below 47·B MHz/T (if higher than the Larmor frequency).

According to a preferred method, the separation of the flow component of the recorded signals from those signal components resulting from movements of the object, e.g. cardiac and respiratory motion, is achieved by using (appropriate) blind and/or semi-blind source separation methods, preferably by using Independent Component Analysis (ICA) or by a machine learning algorithm trained with ground truth data. These methods for separation of signal contributions are well-known. It is also preferred to record a (image) navigator during a reference scan and then determine a combination that provides a norm (e.g. L2) that minimizes the difference between the navigator and the recorded signal (of the response signal or of the flow component).

According to a preferred method, the object is arranged between the generator coil and the RF antennae, wherein at least the signals from the two RF antennae of the RF reception system that are positioned closest to a line parallel to the blood flow intended to be measured is recorded. Since the course of blood vessels in a body is known and the region of interest is known, the appropriate antennae (e.g. coils of a magnetic resonance scanner) can be selected accordingly. Alternatively, it is also possible to arrange antennae appropriately.

It should be noted that it is not necessary for the invention that the object is between the generator coil and the RF antennae, since there can be also recorded a useful response signal if an antenna array is used with an integrated generator coil. The magnetic field emitted by the object can be measured all around the object. This means, the response signals of the modulated magnetic field can be measured at an arbitrary position around the object and not only at a position opposite the generator coil.

According to a preferred method, a modulation of the modulated magnetic field includes a frequency within a frequency range near the Larmor frequency of the hydrogen atoms of the object in an applied magnetic field, preferably within a variance of less than 10 percent deviating from this Larmor frequency.

Alternatively or additionally, a modulation of the modulated magnetic field includes a frequency that is within the receiving frequency bandwidth of the RF antennae used for the measurement, preferably within a frequency range of less than 10 percent deviating from the Eigenfrequency of these RF antennae.

According to a preferred method, after separating the flow component of the recorded signals from those signal components resulting from movements of the object, parameters of the flow are computed, preferably a value of a flux and/or a periodicity. This could be achieved with a preferred computing unit designed to compute parameters of the measured flow, preferably a value of a flux and/or a periodicity. For example, the periodicity can be derived from the shape of the recorded signal over time, the flux can be derived from the delay of recorded signals from different RF antennae.

A preferred system comprises a generator coil designed to generate a modulated magnetic field, wherein the generator coil is preferably positioned inside a magnetic resonance scanner. Additionally, the system preferably comprises a generator unit for producing the modulation signal for the modulated magnetic field.

The generator coil is preferably dumbbell-shaped, e.g. with two circular-shaped parts and an elongated part between these circular-shaped parts. The circular-shaped parts may be small relative to the wavelength of the modulated magnetic field, wherein the parts have preferably a diameter between 5 mm and 10 mm and the elongated part a length between 10 mm and 50 mm. The maximum field strength of the modulated magnetic field lies in the region of mT and is typically below 100 mT and above 1 mT. Especially when receiving units of an MR scanner are used, this field strength should be adjusted such that the received signal level is in the region of the signal levels received at normal MR imaging so that there is no overdrive of the electronics.

A preferred system comprises an RF reception system with at least two RF antennae designed to record response signals of the modulated magnetic field from the object, wherein at least the response signals are measured by the RF antennae. The RF antennae are preferably an RF reception antenna system of a magnetic resonance imaging system.

Other objects and features of the present invention will become apparent from the following detailed descriptions considered in conjunction with the accompanying drawings. It is to be understood, however, that the drawings are designed solely for the purposes of illustration and not as a definition of the limits of the invention.
Figure 1 shows a simplified MRI system with an example for a system according to an embodiment of the invention.
Figure 2 shows a block diagram of the process flow of a preferred method according to the invention.
Figure 3 shows an example of an arrangement of the coils.
Figure 4 outlines an example for a possible measurement without a flow.
Figure 5 outlines an example for a possible measurement with a flow.
Figure 6 shows an example of a flow-measurement.

In the diagrams, like numbers refer to like objects throughout. Objects in the diagrams are not necessarily drawn to scale.

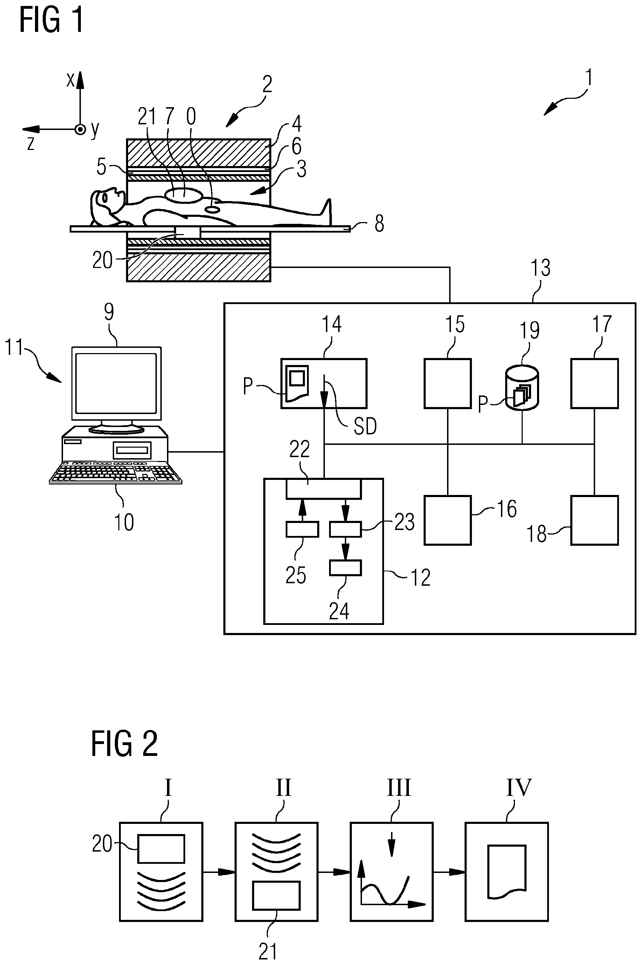

Figure 1 shows a schematic representation of a magnetic resonance imaging system 1 ("MRI system"). The MRI system 1 includes the actual magnetic resonance scanner (data acquisition unit) 2 with an examination space 3 or patient tunnel in which a patient or test person is positioned on a driven bed 8, in whose body the actual examination object is located.

The magnetic resonance scanner 2 is typically equipped with a basic field magnet system 4, a gradient system 6, as well as an RF transmission antenna system 5, and an RF reception antenna system 7. In the shown exemplary embodiment, the RF transmission antenna system 5 is a whole-body coil permanently installed in the magnetic resonance scanner 2, in contrast to which the RF reception antenna system 7 is formed as a plurality of local coils to be arranged on the patient or test subject. In principle, however, the whole-body coil can also be used as an RF reception antenna system, and the local coils can respectively be switched into different operating modes.

In this example, the RF reception antenna system 7 is used as RF antennae 21 according to the invention. Below the object, a generator coil 20 is located. In another example, the RF antennae 21 could be at the position of the whole-body coil (or the whole-body coil can be used if it comprises more than one single coil), and the local coil 7 could be used as generator coil 20.

The basic field magnet system 4 is designed such that a region of Interest ("RoI") can be recorded. Here it is designed in a typical manner so that it generates a basic magnetic field in the longitudinal direction of the patient, i.e. along the longitudinal axis of the magnetic resonance scanner 2 that proceeds in the z-direction. The gradient system 6 typically includes individually controllable gradient coils in order to be able to switch (activate) gradients in the x-direction, y-direction or z-direction independently of one another.

The MRI system 1 shown here is a whole-body system with a patient tunnel into which a patient can be completely introduced. However, in principle the invention can also be used at other MRI systems, for example with a laterally open, C-shaped housing, as well as in smaller magnetic resonance scanners in which only one body part can be positioned.

Furthermore, the MRI system 1 has a central control device 13 that is used to control the MRI system 1. This central control device 13 includes a sequence control unit 14 for measurement sequence control. With this sequence control unit 14, the series of radio frequency pulses ("RF pulses") and gradient pulses can be controlled depending on a selected pulse sequence or, respectively, a series of multiple pulse sequences to acquire magnetic resonance images of the RoI within a measurement session. For example, such a series of pulse sequences can be predetermined within a measurement or control protocol P. Different control protocols P for different measurements or measurement sessions are typically stored in a memory 19 and can be selected by an operator (and possibly modified as necessary) and then be used to implement the measurement.

To output the individual RF pulses of a pulse sequence, the central control device 13 has an RF transmission device 15 that generates and amplifies the RF pulses and feeds them into the RF transmission antenna system 5 via a suitable interface (not shown in detail). To control the gradient coils of the gradient system 6, the control device 13 has a gradient system interface 16. The sequence control unit 14 communicates in a suitable manner with the RF transmission device 15 and the gradient system interface 16 to emit the pulse sequence.

Moreover, the control device 13 has an RF reception device 17 (likewise communicating with the sequence control unit 14 in a suitable manner) in order to acquire magnetic resonance signals (i.e. raw data) for the individual measurements, by which magnetic resonance signals are received in a coordinated manner from the RF reception antenna system 7 within the scope of the pulse sequence.

A reconstruction unit 18 receives the acquired raw data and reconstructs magnetic resonance image data therefrom for the measurements. This reconstruction is typically performed on the basis of parameters that may be specified in the respective measurement or control protocol. For example, the image data can then be stored in a memory 19.

Operation of the central control device 13 can take place via a terminal 11 with an input unit 10 and a display unit 9, via which the entire MRI system 1 can thus also be operated by an operator. MR images can also be displayed at the display unit 9, and measurements can be planned and started by means of the input unit (possibly in combination with the display unit 9), and in particular suitable control protocols can be selected (and possibly modified) with suitable series of pulse sequences as explained above.

The control device 13 comprises a system 12 designed to perform the method according to the invention. This system 12 comprises the following components that may partly appear to be software modules. The system comprises:
A first data-interface 22 designed for sending a signal to a generator coil 20, wherein this signal is designed to generate a (low-power) modulated magnetic field in the generator coil 20. The signal can be produced by a generator unit 25. Since the recording RF antennae have an Eigenfrequency in the range of MHz, the signal should be a periodic MHz-signal.

A second data-interface 22 designed for recording response signals of the modulated magnetic field from the object measured with an RF reception system, wherein at least the response signals are measured by two RF antennae of the RF reception system. Here the first and second data interface are one single data interface designed for bi-directional communication.

A separation unit 23 designed to separate a flow component of the recorded signals from those signal components resulting from cardiac and respiratory motion. Since the measured signals can easily be turned into digital values by an analog-digital-discriminator, the separation could be performed by calculating steps, e.g. by a calculation based on an Independent Component Analysis.

A computing unit 24 that is designed to compute parameters of the measured flow. Here a value for the flux (blood volume per second) and the periodicity of the blood flow is calculated based on the separated data. The flux may be determined from the phase shift between the signals of two (or more) antennae, the volume of flowing blood by the amplitude of a signal.

In this example, the generator coil 20 is part of the magnetic resonance scanner 2. However, it could also be regarded as a part of the system 12. The same is valid for the RF antennae 21. Here the local coils 7 of the magnetic resonance scanner 2 are used as RF antennae 21. However, also the RF antennae 21 could be regarded as a part of the system 12.

The MRI system 1 according to the invention, and in particular the control device 13, can have a number of additional components that are not shown in detail here but are typically present in such systems, for example a network interface in order to connect the entire system with a network and be able to exchange raw data and/or image data or, respectively, parameter maps, but also additional data (for example patient-relevant data or control protocols).

Figure 2 shows a block diagram of the process flow of a preferred method according to the invention

In step I, a modulated magnetic field is generated by a generator coil. The modulation could be following an alternating signal. The frequency of the modulation of the magnetic field is given with the field-strength of the predefined basic magnetic field. This field-strength defines the Larmor frequency that is measured during medical imaging wherein the Eigenfrequency of the RF reception antenna system 7 is designed to meet the Larmor frequency. The frequency of the modulation should be higher or lower than the Larmor frequency but lie within the bandwidth of the RF reception antenna system 7. In this example, the frequency of the modulation may be between 22 MHz and 125 MHz. However, depending on the magnetic field, the frequency may be higher or lower.

In Figure 1, the generating coil 20 is positioned near the object O. This has the advantage that the modulated magnetic field is generated near the region of interest, since the field-strength of the modulated magnetic field decreases with increasing distance d with approximately 1/d³ (Biot-Savart law) .

In step II, response signals of the modulated magnetic field from the object are recorded with an RF reception system, e.g. as shown in Figure 1 the local coil 7. The response signals should be measured by at least two RF antennae of the RF reception system.

The modulated magnetic field applied in step I induces eddy currents in the blood which is a conductive fluid. These eddy currents in turn generate a magnetic field opposing the generated field. When the blood moves due to the heart's pumping activity an additional current is induced due to motion and the local magnetic environment is changed. This can be recorded by the RF antennae.

In step III, the flow component of the recorded signals is separated from those signal components resulting from movements of the object, such as cardiac and respiratory motion. Due to the pumping action of the heart, the signals from the blood flow can be identified and separated from other contributions to the signal by regarding the shape and/or other (statistical) parameters (as e.g. frequency or shape of the distribution) of the signal. For separation from contributions from cardiac and respiratory motion, appropriate blind and/or semi-blind source separation methods can be used such as e.g. ICA.

In step IV, parameters of the blood flow are computed, such as a value of the flux and/or the periodicity of the flow.

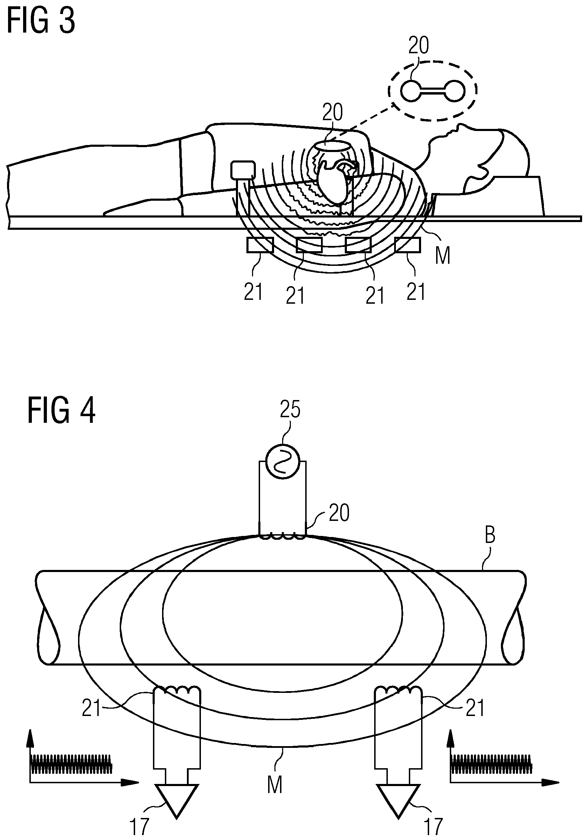

Figure 3 shows an example of an arrangement of the coils. Unlike figure 1, where a local coil 7 was used as RF antenna 21, it is now used as generator coil 20 sending a modulated magnetic field M with a frequency of about 50 MHz to the heart of a patient. The generator coil 20 may be dumbbell-shaped as shown in the dashed example. Here, the movement of the heart should be measured as well as the flow in the Aorta.

Several RF antennae 21, which could be part of the RF reception antenna system 7, measure the response of the modulated magnetic field after it has passed heart and aorta. Here, additional antennae may be used, too, e.g. RF antennae in the body array, since the response signal is emitted by the object in all directions.

The signals recorded with the RF antennae 21 could then be processed in that the movement of the heart is separated from the contribution of the blood flow in the aorta as already explained above.

Figure 4 outlines an example for a possible measurement without a flow. A blood vessel B lies between a generator coil 21 on one side and two RF antennae 20 on the other side. The RF antenna 20 emits a modulated magnetic field M that is created in a generator unit 25 with a frequency in the order of some ten to some hundred MHz, depending on field-strength. The RF antennae 21 measure the modulated magnetic field M passing through the blood vessel B and an RF reception device 17 at each RF antenna forms a measuring signal or measurement data. The measured signal of each RF antenna 21 is shown adjacent the respective RF antenna 21. Since there is no flow in the blood vessel B in this figure, the signals are almost identical (here due to the symmetric setup of the RF antennae 21). Again, it should be noted that the generator coil may e.g. also lie between the blood vessel and the RF antennae, since it is not necessary that the blood vessel lies between the generator coil and the RF antennae.

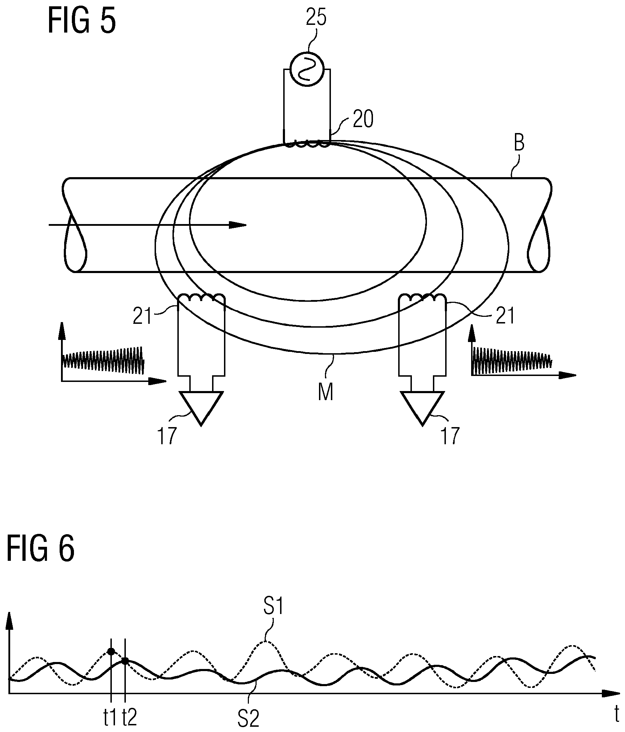

Figure 5 outlines an example for a possible measurement with a flow. The same setup is used as in figure 4. In contrast to figure 4, there is now blood flowing through the blood vessel B causing the magnetic field to slightly deform in comparison to figure 4. Due to this deformation, the measured signals of the RF antennae 21, again shown adjacent the respective RF antenna 21, are different. This difference offers on the one hand a possibility to separate the contribution of the blood flow from contribution of other measurements (that may e.g. form an identical offset in both measurements) and on the other hand a possibility to calculate parameters of the flow itself (e.g. the flux or the periodicity).

Figure 6 shows an example of a flow-measurement, e.g. recorded with the setup of figure 5. The amplitude of the measured signals S1, S2 is drawn against the time t in arbitrary units (e.g. mV). The difference between the amplitudes of the two signals S1, S2 results from different energy input in the RF antennae 21 (see e.g. the different field strength in figure 5 at the position of the RF antennae 21). Thus, the output of the RF antennae 21 depends on their position relative to the generator coil 20 and the flow direction.

It can be seen that the heartbeat modulates the signals S1, S2 so that the pulse frequency of the blood vessel B can be determined. Furthermore, the signal in the second coil is delayed which can be seen by comparing the two points in time t1, t2 pointing to respective peaks of the blood flow. The delay may e.g. be approximately 120ms. Since the position of the RF antennae 21 is well-known, this delay can be used to calculate further parameters as e.g. the velocity of the flow.

Although the present invention has been disclosed in the form of preferred embodiments and variations thereon, it will be understood that numerous additional modifications and variations could be made thereto without departing from the scope of the invention. For the sake of clarity, it is to be understood that the use of "a" or "an" throughout this application does not exclude a plurality, and "comprising" does not ex-elude other steps or elements. The mention of a "unit" or a "device" does not preclude the use of more than one unit or device.

## Claims

1. A method for measuring blood flow comprising the following steps:
- generating a modulated magnetic field (M) that is a pilot tone signal by a generator coil (20),
- recording response signals (S1, S2) of the modulated magnetic field (M) from an object (O) with an RF reception system (7, 21, 17), wherein the response signals (S1, S2) are measured by at least two RF antennae (21) of the RF reception system (7, 21, 17),
- separating a flow component of the recorded response signals (S1, S2) from those signal components resulting from movements of the object (O), wherein a flow movement is differentiated from other disturbing influences by a specific frequency and signal profile.

2. The method according to claim 1, wherein the generator coil (20) is embedded in a magnetic resonance scanner (2) of a magnetic resonance imaging system (1), preferably near or in an RF reception antenna system (7) of the magnetic resonance scanner (2).

3. The method according to one of the preceding claims,
wherein the RF reception system (7, 21, 17) used for recording the response signals of the modulated magnetic field (M) is the RF reception system (7, 17) of a magnetic resonance imaging system, wherein preferably the response signals (S1, S2) of the modulated magnetic field (M) are recorded by RF antennae (21) from the RF reception antenna system (7) of the magnetic resonance scanner (2),
wherein preferably more than three RF antennae (21) are used for measurement.

4. The method according to one of the preceding claims,
wherein the separation of the flow component of the recorded signals (S1, S2) from those signal components resulting from movements of the object (O), especially from cardiac and respiratory motion, is achieved by using blind and/or semi-blind source separation methods, preferably by using Independent Component Analysis (ICA) or by using a machine learning algorithm trained with ground truth data.

5. The method according to one of the preceding claims,
wherein the object (O) is arranged between the generator coil (20) and the RF antennae (21), wherein at least the signals from the two RF antennae (21) of the RF reception system positioned closest to a line parallel to the blood flow intended to be measured is recorded.

6. The method according to one of the preceding claims, wherein a modulation of the modulated magnetic field (M) includes a frequency
- within a frequency range which corresponds approximately to the Larmor frequency of the hydrogen atoms of the object in an applied magnetic field (M), preferably within a variance of less than 10 percent deviating from this Larmor frequency
and/or
- within a receiving frequency bandwidth of the RF antennae (21) used for the measurement, preferably within a frequency range of less than 10 percent deviating from the Eigenfrequency of these RF antennae (21).

7. The method according to one of the preceding claims,
wherein after separating the flow component of the recorded signals (S1, S2) from those signal components resulting from movements of the object (O), parameters of the flow are computed, preferably a value of a flux and/or a periodicity.

8. A system for measuring blood flow comprising:
- a first data-interface (22) designed for sending a signal to a generator coil (20), wherein this signal is designed to generate a modulated magnetic field (M) that is a pilot tone signal in the generator coil (20),
- a second data-interface (22) designed for recording response signals (S1, S2) of the modulated magnetic field (M) from an object (O) measured with an RF reception system (7, 21, 17), wherein at least the response signals (S1, S2) are measured by two RF antennae (21) of the RF reception system (7, 21, 17),
- a separation unit (23) designed to separate a flow component of the recorded response signals (S1, S2) from those signal components resulting from movements of the object, wherein a flow movement is differentiated from other disturbing influences by a specific frequency and signal profile.

9. The system according to claim 8, comprising a computing unit (24) designed to compute parameters of the measured flow, preferably a value of a flux and/or a periodicity.

10. The system according to claim 8 or 9, comprising a generator coil (20) designed to generate a modulated magnetic field (M), wherein the generator coil (20) is preferably positioned inside a magnetic resonance scanner (2), and/or the system preferably comprises a generator unit (25) for producing the modulation signal for the modulated magnetic field (M) .

11. The system according to one of claims 8 to 10, comprising an RF reception system (7, 21, 17) with at least two RF antennae (21) designed to record response signals (S1, S2) of the modulated magnetic field (M) from the object (O), wherein at least the response signals (S1, S2) are measured by the RF antennae (21),
wherein the RF antennae (21) are preferably an RF reception antenna system (5) of a magnetic resonance imaging system (1) .

12. A control device for controlling a magnetic resonance imaging system comprising a system according to one of claims 8 to 11 and/or designed to perform a method according to any of claims 1 to 7.

13. A magnetic resonance imaging system comprising a control device according to claim 12.

14. A computer program product comprising a computer program that is directly loadable into a system or a control device for a magnetic resonance imaging system, which comprises program elements for performing the steps of the method according to any of claims 1 to 7 when the computer program is executed by the system or the control device.

15. A computer-readable medium on which program elements are stored that can be read and executed by a computer unit in order to perform the steps of the method according to any of claims 1 to 7 when the program elements are executed by the computer unit.

## Patentansprüche

1. Verfahren zum Messen des Blutflusses, umfassend die folgenden Schritte:
- Generieren eines modulierten Magnetfelds (M), das ein Pilottonsignal ist, durch eine Generatorspule (20),
- Aufzeichnen von Antwortsignalen (S1, S2) des modulierten Magnetfelds (M) von einem Objekt (O) mit einem HF-Empfangssystem (7, 21, 17), wobei die Antwortsignale (S1, S2) durch mindestens zwei HF-Antennen (21) des HF-Empfangssystems (7, 21, 17) gemessen werden,
- Trennen einer Flusskomponente der aufgezeichneten Antwortsignale (S1, S2) von jenen Signalkomponenten, die aus Bewegungen des Objekts (O) resultieren, wobei eine Flussbewegung durch ein spezifisches Frequenz- und Signalprofil von anderen störenden Einflüssen differenziert wird.

2. Verfahren nach Anspruch 1, wobei die Generatorspule (20) in einen Magnetresonanzscanner (2) eines Magnetresonanzbildgebungssystems (1) eingebettet ist, vorzugsweise nahe oder in einem HF-Empfangsantennensystem (7) des Magnetresonanzscanners (2).

3. Verfahren nach einem der vorhergehenden Ansprüche, wobei das HF-Empfangssystem (7, 21, 17), das zum Aufzeichnen der Antwortsignale des modulierten Magnetfelds (M) verwendet wird, das HF-Empfangssystem (7, 17) eines Magnetresonanzbildgebungssystems ist, wobei vorzugsweise die Antwortsignale (S1, S2) des modulierten Magnetfelds (M) durch HF-Antennen (21) von dem HF-Empfangsantennensystem (7) des Magnetresonanzscanners (2) aufgezeichnet werden,
wobei vorzugsweise für die Messung mehr als drei HF-Antennen (21) verwendet werden.

4. Verfahren nach einem der vorhergehenden Ansprüche, wobei die Trennung der Flusskomponente der aufgezeichneten Signale (S1, S2) von jenen Signalkomponenten, die aus Bewegungen des Objekts (O) resultieren, insbesondere durch kardiale und respiratorische Bewegung, durch Verwendung von blinden und/oder halbblinden Quellentrennungsverfahren erreicht wird, vorzugsweise durch Verwendung von unabhängiger Komponentenanalyse (ICA) oder durch Verwendung eines Maschinenlernalgorithmus, der mit Ground-Truth-Daten trainiert wurde.

5. Verfahren nach einem der vorhergehenden Ansprüche, wobei das Objekt (O) zwischen der Generatorspule (20) und den HF-Antennen (21) angeordnet ist, wobei mindestens die Signale von den beiden HF-Antennen (21) des HF-Empfangssystems, die einer Leitung am nächsten positioniert sind, welche parallel zu dem zu messenden Blutfluss ist, aufgezeichnet werden.

6. Verfahren nach einem der vorhergehenden Ansprüche, wobei eine Modulation des modulierten Magnetfelds (M) eine Frequenz einschließt, die
- innerhalb eines Frequenzbereichs liegt, der ungefähr der Larmorfrequenz der Wasserstoffatome des Objekts in einem angelegten Magnetfeld (M) entspricht, vorzugsweise innerhalb einer Varianz von weniger als 10 Prozent Abweichung von dieser Larmorfrequenz,
und/oder
- innerhalb einer Empfangsfrequenzbandbreite der HF-Antenne (21) liegt, die für die Messung verwendet wird, vorzugsweise innerhalb eines Frequenzbereichs von weniger als 10 Prozent Abweichung von der Eigenfrequenz dieser HF-Antennen (21).

7. Verfahren nach einem der vorhergehenden Ansprüche, wobei nach dem Trennen der Flusskomponente der aufgezeichneten Signale (S1, S2) von jenen Signalkomponenten, die aus Bewegungen des Objekts (O) resultieren, Parameter des Flusses berechnet werden, vorzugsweise ein Wert eines Flux und/oder einer Periodizität.

8. System zum Messen des Blutflusses, umfassend:
- eine erste Datenschnittstelle (22), die zum Senden eines Signals an eine Generatorspule (20) konzipiert ist, wobei dieses Signal konzipiert ist, um ein moduliertes Magnetfeld (M) zu generieren, das ein Pilottonsignal in der Generatorspule (20) ist,
- eine zweite Datenschnittstelle (22), die zum Aufzeichnen von Antwortsignalen (S1, S2) des modulierten Magnetfelds (M) von einem Objekt (O) konzipiert ist, das mit einem HF-Empfangssystem (7, 21, 17) gemessen wird, wobei mindestens die Antwortsignale (S1, S2) durch mindestens zwei HF-Antennen (21) des HF-Empfangssystems (7, 21, 17) gemessen werden,
- eine Trenneinheit (23), die zum Trennen einer Flusskomponente der aufgezeichneten Antwortsignale (S1, S2) von jenen Signalkomponenten konzipiert ist, die aus Bewegungen des Objekts resultieren, wobei eine Flussbewegung durch ein spezifisches Frequenz- und Signalprofil von anderen störenden Einflüssen differenziert wird.

9. System nach Anspruch 8, umfassend eine Recheneinheit (24), die konzipiert ist, um Parameter des gemessenen Flusses zu berechnen, vorzugsweise einen Wert eines Flux und/oder einer Periodizität.

10. System nach Anspruch 8 oder 9, umfassend eine Generatorspule (20), die konzipiert ist, um ein moduliertes Magnetfeld (M) zu generieren, wobei die Generatorspule (20) vorzugsweise innerhalb eines Magnetresonanzscanners (2) positioniert ist und/oder das System vorzugsweise eine Generatoreinheit (25) zum Produzieren des Modulationssignals für das modulierte Magnetfeld (M) umfasst.

11. System nach einem der Ansprüche 8 bis 10, umfassend ein HF-Empfangssystem (7, 21, 17) mit mindestens zwei HF-Antennen (21), die konzipiert sind, um Antwortsignale (S1, S2) des modulierten Magnetfelds (M) von dem Objekt (O) aufzuzeichnen, wobei mindestens die Antwortsignale (S1, S2) durch die HF-Antennen (21) gemessen werden,
wobei die HF-Antennen (21) vorzugsweise ein HF-Empfangsantennensystem (5) eines Magnetresonanzbildgebungssystems (1) sind.

12. Steuervorrichtung zum Steuern eines Magnetresonanzbildgebungssystems, umfassend ein System gemäß einem der Ansprüche 8 bis 11 und/oder konzipiert, um ein Verfahren gemäß einem der Ansprüche 1 bis 7 durchzuführen.

13. Magnetresonanzbildgebungssystem, umfassend eine Steuervorrichtung nach Anspruch 12.

14. Computerprogrammprodukt, umfassend ein Computerprogramm, das direkt in ein System oder eine Steuervorrichtung für ein Magnetresonanzbildgebungssystem ladbar ist, das Programmelemente zum Durchführen der Schritte des Verfahrens gemäß einem der Ansprüche 1 bis 7 umfasst, wenn das Computerprogramm durch das System oder die Steuervorrichtung ausgeführt wird.

15. Computerlesbares Medium, auf dem Programmelemente gespeichert sind, die durch eine Computereinheit gelesen und ausgeführt werden können, um die Schritte des Verfahrens nach einem der Ansprüche 1 bis 7 durchzuführen, wenn die Programmelemente durch die Computereinheit ausgeführt werden.

## Revendications

1. Procédé de mesure du débit sanguin comprenant les stades suivants :
- production d'un champ (M) magnétique modulé, qui est un signal audio pilote par une bobine (20) de générateur,
- enregistrement de signaux (S1, S2) de réaction du champ (M) magnétique modulé à partir d'un objet (O) par un système (7, 21, 17) de réception RF, les signaux (S1, S2) de réaction étant mesurés par au moins deux antennes (21) RF du système (7, 21, 17) de réception RF,
- séparation d'une composante de débit des signaux (S1, S2) de réaction enregistrés des composantes de signal provenant de déplacements de l'objet (O), un déplacement de débit étant différencié d'autres influences perturbatrices par une fréquence et un profil de signal spécifique.

2. Procédé suivant la revendication 1, dans lequel la bobine (20) du générateur est incorporée à un scanneur de résonnance magnétique d'un système (1) d'imagerie par résonnance magnétique, en étant de préférence proche ou dans un système (7) d'antenne de réception RF du scanner (2) par résonnance magnétique.

3. Procédé suivant l'une des revendications précédentes, dans lequel le système (7, 21, 17) de réception RF utilisé pour enregistrer les signaux de réaction du champ (M) magnétique modulé est le système (7, 17) de réception RF d'un système d'imagerie par résonnance magnétique, les signaux (S1, S2) de réaction du champ (M) magnétique modulé étant de préférence enregistrés par des antennes (21) RF provenant du système (7) d'antenne de réception RF du scanner (2) par résonnance magnétique,
dans lequel on utilise de préférence plus que trois antennes (21) RF pour la mesure.

4. Procédé suivant l'une des revendications précédentes, dans lequel on obtient la séparation de la composante de débit des signaux (S1, S2) enregistrés des composantes de signal provenant de déplacements de l'objet (O), en particulier du mouvement cardiaque et respiratoire, en utilisant des procédés de séparation à la source aveugle et/ou semi-aveugle, de préférence en utilisant l'Independent Component Analysis (ICA) ou en utilisant un algorithme d'apprentissage automatique ayant subi un apprentissage par des données de vérité de base.

5. Procédé suivant l'une des revendications précédentes, dans lequel on dispose l'objet (O) entre la bobine (20) du générateur et les antennes (21) RF, au moins les signaux, provenant des deux antennes (21) RF du système de réception (RF), placées le plus près d'une ligne parallèle au débit sanguin que l'on a l'intention de mesurer, étant enregistrés.

6. Procédé suivant l'une quelconque des revendications précédentes, dans lequel une modulation du champ (M) magnétique modulé inclut une fréquence
- dans une plage de fréquence, qui correspond approximativement à la fréquence de Larmor des atomes d'hydrogène de l'objet dans un champ (M) magnétique appliqué, de préférence dans une variance de moins de 10% s'écartant de cette fréquence de Larmor
et/ou
- dans une largeur de bande de fréquence de réception des antennes (21) RF utilisées pour la mesure, de préférence dans une plage de fréquence s'écartant de moins de 10% de la fréquence propre de ces antennes (21) RF.

7. Procédé suivant l'une des revendications précédentes, dans lequel, après séparation de la composante de débit des signaux (S1, S2) enregistrés des composantes de signal provenant des déplacements de l'objet (O), on calcule des paramètres du débit, de préférence une valeur d'un flux et/ou une périodicité.

8. Système de mesure d'un débit sanguin comprenant :
- une première interface (22) de données, conçue pour envoyer un signal à une bobine (20) de générateur, ce signal étant conçu pour produire un champ (M) magnétique modulé, qui est un signal audio pilote dans la bobine (20) du générateur,
- une deuxième interface (22) de données, conçue pour enregistrer des signaux (S1, S2) de réaction du champ (M) magnétique modulé à partir d'un objet (O) mesurés par un système (7, 21, 17) de réception RF, les signaux (S1, S2) de réaction étant mesurés au moins par deux antennes (21) RF du système (7, 21, 17) de réception RF,
- une unité (23) de séparation, conçue pour séparer une composante de débit des signaux (S1, S2) de réaction enregistrés des composantes de signal provenant de déplacements de l'objet, un déplacement de débit étant différencié d'autres influences perturbatrices par une fréquence et un profil de signal spécifique.

9. Système de mesure suivant la revendication 8, comprenant une unité (24) informatique conçue pour calculer des paramètres du débit mesuré, de préférence une valeur d'un flux et/ou une périodicité.

10. Système de mesure suivant la revendication 8 ou 9, comprenant une bobine (20) de générateur, conçue pour produire un champ (M) magnétique modulé, la bobine (20) du générateur étant placée de préférence à l'intérieur d'un scanner (2) par résonnance magnétique et/ou le système comprend de préférence une unité (25) de générateur pour produire le signal de modulation pour le champ (M) magnétique modulé.

11. Système de mesure suivant l'une des revendications 8 à 10, comprenant un système (7, 21, 17) de réception RF ayant au moins deux antennes (21) RF conçues pour enregistrer des signaux (S1, S2) de réaction du champ (M) magnétique modulé à partir de l'objet (O), au moins les signaux (S1, S2) de réaction étant mesurés par les antennes (21) RF,
dans lequel les antennes (21) RF sont de préférence un système (5) d'antennes de réception RF d'un système (1) d'imagerie par résonnance magnétique.

12. Dispositif de commande pour commander un système d'imagerie par résonnance magnétique comprenant un système suivant l'une des revendications 8 à 11 et/ou conçu pour effectuer un procédé suivant l'une des revendications 1 à 7.

13. Système d'imagerie par résonnance magnétique, comprenant un dispositif de commande suivant la revendication 12.

14. Produit de programme d'ordinateur, comprenant un programme d'ordinateur, qui peut être chargé directement dans un système ou dans un dispositif de commande d'un système d'imagerie par résonnance magnétique, qui comprend des éléments de programme pour effectuer les stades du procédé suivant l'une quelconque des revendications 1 à 7 lorsque le programme d'ordinateur est exécuté par le dispositif de commande.

15. Support déchiffrable par ordinateur, sur lequel sont mis en mémoire des éléments de programme, qui peuvent être lus et exécutés par une unité informatique, afin d'effectuer les stades du procédé suivant l'une quelconque des revendications 1 à 7 lorsque les éléments de programme sont exécutés par l'unité informatique.
